# EUROPEAN PATENT SPECIFICATION

(11) **EP 1 959 828 B1**
(45) Date of publication and mention of the grant of the patent: **27.02.2013**
(21) Application number: 06829510.4
(22) Date of filing: 12.12.2006
(51) Int. Cl.: A61B 5/053

(54) **Diagnosing of a diseased condition of the skin using impedance**
Diagnose eines Krankheitszustands der Haut mit Impedanz
Appareil médical pour le diagnostic d'affections de la peau à l'aide de mesures d'impédance

(30) Priority: 14.12.2005 SE 0502761; 14.12.2005 US 302498
(43) Date of publication of application: 27.08.2008
(73) Proprietor: SciBase AB, 111 40 Stockholm (SE)
(72) Inventor: OLLMAR, Stig, S-141 41 Huddinge (SE); NICANDER, Ingrid, S-136 73 Huddinge (SE); BIRGERSSON, Ulrik, S-118 59 Stockholm (SE); CORMAN, Thierry, S-192 72 Sollentuna (SE); ÅBERG, Peter, S-122 46 Enskede (SE)
(74) Representative: Loqvist, Gabriel Mathias
(86) International application number: PCT/EP2006/011924
(87) International publication number: WO 2007/068433

(56) References cited:
- EP-A- 1 437 091
- WO-A-01/52731
- US-A1- 2003 050 548
- US-A1- 2003 078 482
- US-B1- 6 743 211

## Description

### FIELD OF THE INVENTION

The present invention generally relates to the field of diagnosis of biological conditions, and in particular to a medical apparatus for non-invasively diagnosing a diseased condition of the skin of a subject, particularly the presence of skin cancer, e.g. basal cell carcinoma or malignant melanoma, a squamous cell carcinoma or precursors thereof, using skin impedance measurements.

### BACKGROUND OF THE INVENTION

Basal cell carcinoma (BCC) is the most common skin cancer. Its incidence is increasing in many countries throughout the world, for example, in Sweden. Long-term immunosuppression, e.g. after allogeneic organ transplantation, increases the risk of developing BCC and other skin tumours. This is also the case following exposure to ultraviolet light or ionizing radiation. There seems to be no apparent genetic connection and in many patients no other predisposing factors are found. However, clinical diagnosis of skin tumours can prove difficult even for experienced dermatologists, especially in the case of pigmented lesions. In the clinic there is a need for a diagnostic aid besides the established naked eye in combination with skin biopsies for histological examination.

Non-invasive methods of making biological determinations, such as clinical diagnosis of skin tumours, are generally desirable over invasive techniques that involve the taking of samples. Non-invasive techniques can be more convenient, e.g. less painful, involve less risk of infection etc. Accordingly, a number of non-invasive techniques for making biological determinations have been proposed:

| | | | |
|---|---|---|---|
| US 5,036,861 | August 6, 1991 | Sembrowich *et al.* | |
| US 5,115,133 | May 19, 1992 | Knudson | |
| US 5,146,091 | September 8, 1992 | Knudson | |
| US 5,197,951 | January 19, 1993 | Knudso | |
| US 5,222,496 | June 29, 1993 | Clarke *et al.* | |
| PCT/US94/08816 | WO95/04496 | February 16, 1995 | Solid State Farms, Stark |
| US 5,433,197 | | July 18, 1995 | |
| PCT/US97/13267 | WO 98/04190 | February 5, 1998 | Dermal Therapy (Barbados) Inc. |
| PCT/US98/02037 | WO 99/39627 | August 12, 1999 | Dermal Therapy (Barbados) Inc. |
| PCT/IB00/01464 | WO 01/26338 | October 13, 2000 | Süsstrunk, *et al.* |

However, all of the above-mentioned documents disclose techniques for evaluating blood glucose levels and are consequently not suitable for diagnosing a diseased condition of the skin of a subject, particularly the presence of skin cancer, e.g. basal cell carcinoma.

Electrical impedance has been found to constitute a very sensitive indicator of minute changes in organic and biological material and especially tissues such as mucous membranes, skin and integuments of organs and, hence, provides an effective tool for non-invasive measurements of variations in structural properties of the tissue. In PCT/SE91/00703 (publication number WO-A-92/06634) a device for non-invasive measurement of electrical impedance of organic and biological material is disclosed, including a probe comprising a number of electrodes arranged in a concentric ring system. The electrodes are driven from a control unit in such a way that the electric current path defining the actual tissue under test is dependent upon a control signal, is pressed towards the surface of the body part under test. By varying the control signal it is possible to select the region under test. Two rings supply voltage and the relation between the two will generate a virtual projection point located between the two rings. By adjusting the voltage relation between the two injection points the virtual injection point can be moved back and forth and hence the depth penetration in the tissue is selectable. However, human skin is a complex, heterogeneous, and anisotropic multilayer structure with electronically non-linear properties. The most non-linear properties are located to the Stratum Corneum (the outermost layer of epidermis). Therefore, there is information in all depth measurements that cannot be calculated by interpolation or extrapolation from two depths although the different depths are highly correlated. However, the device disclosed in PCT/SE91/00703 is impaired with a severe drawback in that it cannot deliver reliable results regarding tissue variation in skin layers beneath stratum corneum where, for example, skin cancer and allergic reactions manifest. This is due to the fact that non-invasive electrical impedance spectra of skin are dominated by the dielectric properties of the Stratum Corneum, especially at low frequencies. The stratum corneum has properties (a large and broad so called alpha dispersion) that may lead to that responses from underlying viable skin layers is confounded with responses from stratum corneum, thus diluting the clinically relevant information from the viable skin. This makes it difficult and unpredictable to assess electrical impedance phenomena that manifest below the stratum corneum using the probe disclosed in PCT/SE91/00703.

WO 01/52731 discloses a medical electrode for sensing electric bio-potentials created within the body of a living subject. The electrode comprises a number of micro-needles adapted to penetrate the skin. The micro-needles are long enough to reach the stratum germinativum and are electrically conductive on their surface and connected to each other to form an array. According to WO 01/52731, the micro-needles are "nail-like", i.e. they have stem having a substantially circular cross-section with a constant or a gradually decreasing diameter and a tip-portion with a substantially spherical or needle-shaped tip. In EP 1 437 091 an apparatus for diagnosis of biological conditions using impedance measurements of organic and biological material is disclosed. The apparatus comprises a probe including a plurality of electrodes, where each electrode is provided with a number of micro-needles each having a length being sufficient to penetrate stratum corneum. That known medical apparatus is adapted to initiate an impedance measurement session including passing an electrical current through the electrodes to obtain values of skin impedance, and use reference data to determine whether the obtained impedance values indicate the diseased condition. Furthermore, this known apparatus comprises three electrodes, wherein the micro-needles of a first electrode and a second electrode being laterally spaced apart a first distance from each other and the micro-needles of the first and a third electrode being laterally spaced apart a second distance from each other, said apparatus being adapted to gradually change a proportion of a potential between the first and the second electrode and the first and the third electrode to obtain first and second values of skin impedance or a mixture of values of skin impedance between values obtained using the first and second electrode and values obtained using the first and third electrode. Finally, in the apparatus according to document EP 1 437 091 each electrode comprises a base substrate, said micro-needles being integrally formed with said substrate and arranged in a laterally spaced relationship apart from each other and having a length being sufficient to penetrate the stratum corneum. The micro-needles according to EP 1 437 091 are also "nail-like", i.e. they have stem having a substantially circular cross-section with a constant or a gradually decreasing diameter and a tip-portion with a substantially spherical or needle-shaped tip.

However, practical experience has shown that electrodes furnished with micro-needles according to the conventional technique requires a substantial force or requires a significant manipulating, for example, tilting the electrode to and fro, in order to obtain a proper penetration of the micro-needles into the stratum germinativum. This may be uncomfortable and cumbersome for the patient as well as for the operator, e.g. a physician or a nurse, performing the test. Moreover, this may also require that the needles are designed with a sufficient strength in order to avoid the possibility of needle breakage during use, e.g. by increasing its size.

Thus, there is a need of an improved apparatus for a non-invasively diagnosing a diseased condition of the skin of a subject, particularly the presence of skin cancer, e.g. basal cell carcinoma or malignant melanoma, a squamous cell carcinoma or precursors thereof.

### BRIEF DESCRIPTION OF THE INVENTION

Thus, an object of the present invention is to provide an improved apparatus for diagnosing a diseased condition of the skin of a subject, particularly the presence of skin cancer, e.g. basal cell carcinoma or malignant melanoma, a squamous cell carcinoma or precursors thereof in an accurate and reliable way.

It is another object of the present invention to provide an improved apparatus for diagnosing a diseased condition of the skin of a subject having micro-needles with good penetration ability.

It is a further object of the present invention to provide an improved apparatus for diagnosing a diseased condition of the skin of a subject having micro-needles with a high structural strength.

It is yet another object of the present invention to provide an improved apparatus for diagnosing a diseased condition of the skin of a subject having micro-needles with good electrical characteristics.

These and other objects are achieved according to the present invention by providing a medical apparatus having the features defined in the independent claim. Preferable embodiments of the invention are characterised by the dependent claims.

The claimed apparatus comprises an electrically conducting probe including a plurality of electrodes, each electrode comprising a plurality of micro-needles, wherein the probe is adapted to be placed against a surface of the subject such that the micro-needles penetrate the stratum corneum, wherein the medical apparatus is adapted to initiate an impedance measurement session including passing an electrical current through the electrodes to obtain values of skin impedance, and use reference data to determine whether the obtained impedance values indicate the diseased condition. Each electrode comprises a base substrate, the micro-needles being integrally formed with the substrate and arranged in a laterally spaced relationship apart from each other and having a length being sufficient to penetrate the stratum corneum, the micro-needles being arranged with an at least partially oblique shape.

Thus, the invention is based on the insight that the non-invasive electrical impedance spectra of skin are dominated by the dielectric properties of the Stratum Corneum, especially at low frequencies. The stratum corneum has properties (a large and broad so called alpha dispersion) that may lead to that responses from underlying viable skin layers is confounded with responses from stratum corneum, thus diluting the clinically relevant information from the viable skin. In particular, the present invention is based on the design of the micro-needles with respect to a high degree of structure strength, good electric performance (e.g. with respect to conduction performance), and penetration ability.

This invention provides several advantages. One advantage is that electrical impedance phenomena that manifest below the stratum corneum can be assessed in a reliable manner using the present invention. Thereby, by using the present invention, a diseased condition of the skin of a subject, particularly the presence of skin cancer, e.g. basal cell carcinoma or malignant melanoma, a squamous cell carcinoma or precursors thereof can be diagnosed in an accurate and reliable way.

Another advantage is that the electrodes according to the present invention require less pressure than conventional electrodes to penetrate the stratum corneum. Thereby, the electrodes according to the invention is less dependent on the applied pressure, and hence less operator dependent. This also entails significantly less inconvenience for the patient or person subjected for the test in comparison with tests performed with the conventional electrodes.

A further advantage is that the micro-needles of the electrodes according to the present invention has a high degree of structural strength, that is, the needles are durable.

Yet another advantage is that the contact surface of a micro-needle according to the present invention against the skin of the subject can be made large leading to a good conduction between the needle and the stratum corneum, which, in turn, entails that the impedance can be measured with a high degree of accuracy and reliability.

Furthemore, in the claimed apparatus each of the micro-needles is arranged with a substantially triangular cross-section. This design has been found to have a high degree of structure strength and a high penetration ability. Moreover, it has a large contact surface against the skin of the subject. This leads to a good conduction between the needle and the stratum corneum, which, in turn, entails that the impedance can be measured with a high degree of accuracy and reliability.

Furthermore, the micro-needles of a first electrode and a second electrode is laterally spaced apart a first distance from each other and the micro-needles of the first and a third electrode is laterally spaced apart a second distance from each other, wherein a proportion of a potential between the first and the second electrode and the first and the third electrode is changed stepwise or gradually to obtain first and second values of skin impedance or a mixture of values of skin impedance between values obtained using the first and second electrode and values obtained using the first and third electrode. Thereby, the depth at which measurements are being performed can be selected in an accurate and reliable way.

In addition, the present invention may be used for measuring and/or monitoring and/or detecting biological conditions, for example, changes of skin properties of a subject, or changes of tissue properties of a subject. Moreover, the present invention may also be used for measuring and/or monitoring and/or detecting biological conditions of tissue of an organ, such as a kidney.

The features that characterize the invention, both as to organization and to method of operation, together with further objects and advantages thereof, will be better understood from the following description used in conjunction with the accompanying drawings. It is to be expressly understood that the drawings is for the purpose of illustration and description and is not intended as a definition of the limits of the invention. These and other objects attained, and advantages offered, by the present invention will become more fully apparent as the description that now follows is read in conjunction with the accompanying drawings.

### BRIEF DESCRIPTION OF THE DRAWINGS

In the following detailed description, reference will be made to the accompanying drawings, of which:
Fig. 1a a schematically shows a side view of a micro-needle for use in an electrode not falling within the scope of the present invention;
Fig. 1b schematically shows a top view of the micro-needle shown in Fig. 1a;
Fig. 2a schematically shows a side view of an embodiment of a micro-needle for use in an electrode of the apparatus according to the present invention;
Fig. 2b schematically shows a top view of the micro-needle shown in Fig. 2a;
Fig. 3a schematically shows a side view of an etched wafer during a manufacturing of micro-needles;
Fig. 3b schematically shows a top view of the etched wafer in Fig. 3a during a manufacturing of micro-needles;
Fig. 4a schematically shows a side view of an etched wafer provided with a masking layer;
Fig. 4b schematically shows a side view of the needle resulting from the masking layer in Fig. 4a;
Fig. 5a schematically shows a top view of a ridge produced by an anisotropic etching process provided with a first mask configuration;
Fig. 5b schematically shows a top view of a ridge produced by an anisotropic etching process provided with a second mask configuration;
Fig. 5c schematically shows a top view of a ridge produced by an anisotropic etching process provided with a third mask configuration;
Fig. 5d schematically shows a side view of a needle obtained by the first mask configuration;
Fig. 5e schematically shows a side view of a needle obtained by the second mask configuration;
Fig. 5f schematically shows a side view of a needle obtained by the third mask configuration;
Fig. 6a schematically shows a side view of an etched wafer provided with a masking layer;
Fig. 6b schematically shows a side view of the needle resulting from the masking layer in Fig. 6a;
Fig. 7a shows an embodiment of a probe including electrodes furnished with micro-needles according to the present invention arranged on a removable cap;
Fig. 7b shows a procedure for attaching the removable cap comprising electrodes furnished with micro-needles according to the present invention on the probe shown in Fig. 7a;
Fig. 7c shows a procedure for attaching the removable cap comprising electrodes furnished with micro-needles according to the present invention on the probe shown in Fig. 7a;
Fig. 7d shows a procedure for attaching the removable cap comprising electrodes furnished with micro-needles according to the present invention on the probe shown in Fig. 7a;
Fig. 8a shows a cross-section of probe including electrodes furnished with micro-needles according to the present invention arranged on a removable cap;
Fig. 8b is an enlarged view of cap shown in Fig. 8a in cross-section;
Fig. 9a shows a diagram of the results obtained by measurements at a first site of normal (unaffected skin), i.e. a reference site, using the probe according to the present invention; and
Fig. 9b shows a diagram of the results obtained by measurements at a second site of diseased skin using the probe according to the present invention.

### DETAILED DESCRIPTION OF THE INVENTION

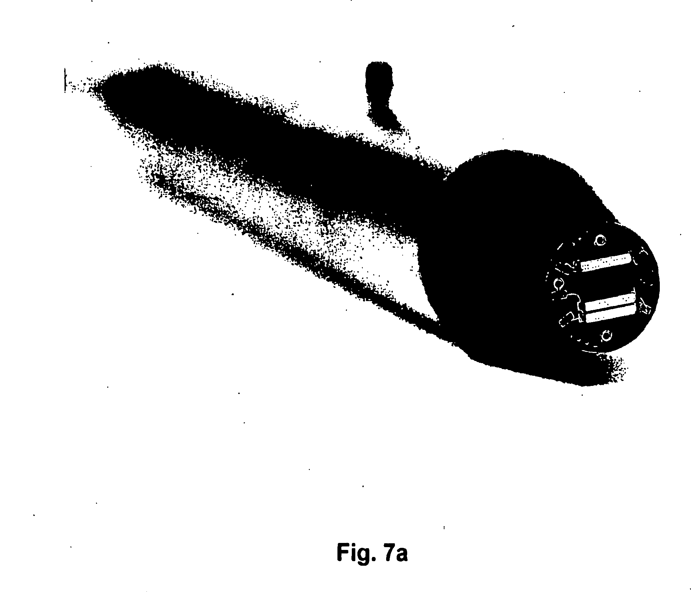

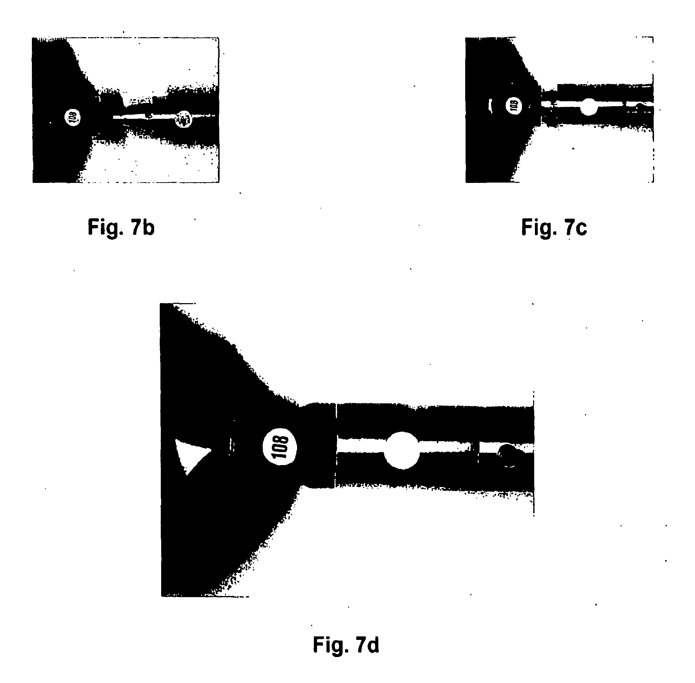

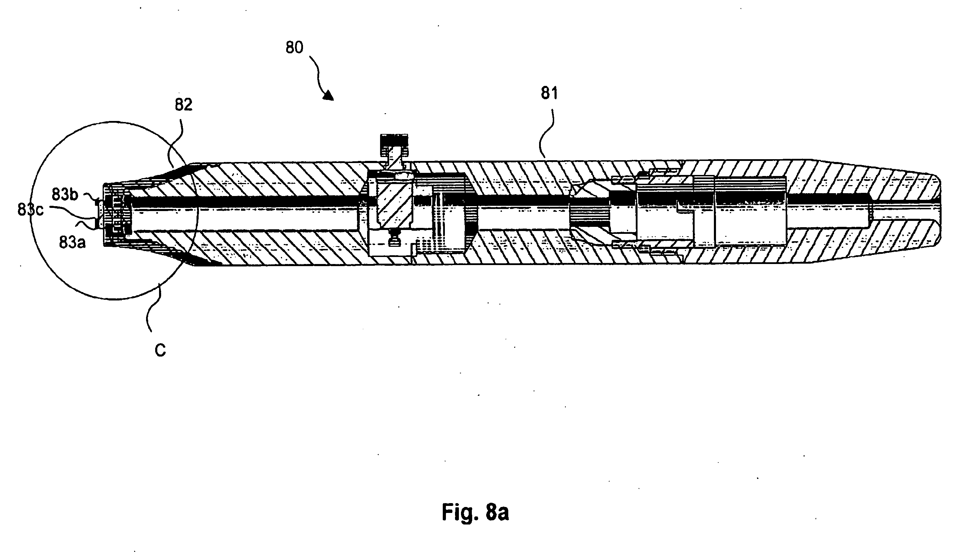

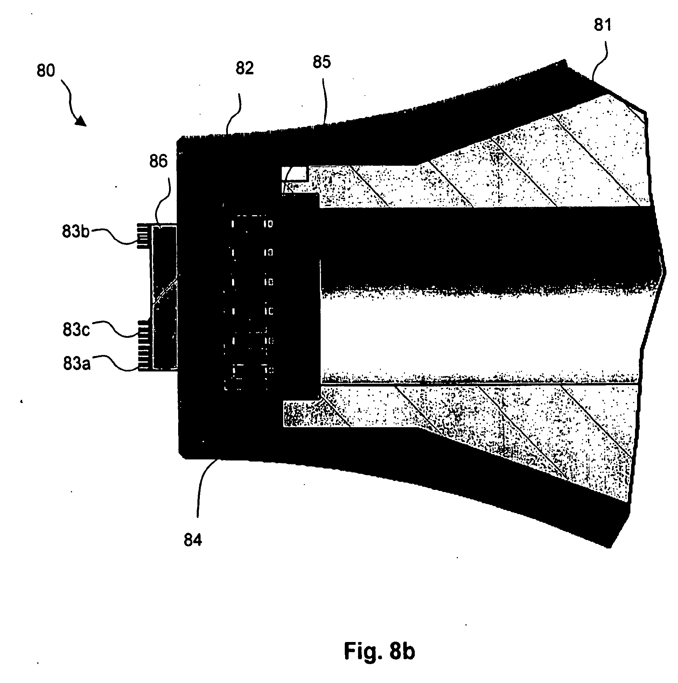

The probe according to the present invention includes a number of electrodes, at least three, and in the present invention each electrode is provided with at least one micro-needle, thereby forming a micro-needled surface, which permits measurements to be made at a variety of skin depths. The probe includes three rectangular areas or bars, each bar containing an array of 57 (19 x 3) micro-needles. Each bar is I mm wide and 5 mm long. The distance between the closest bars is 0.2 mm, and the distance between the second and the third bars is 1.8 mm. The active part of the probe is thus about 5 x 5 mm. Each micro-needle has a length of approximately 100 micrometer, as measured from its base, and a thickness of approximately 30 micrometer. The micro-needles are preferably made of silicon and covered with gold having a thickness of approximately 2 micrometer. Any material comprising a conductive surface with similar dimensions would work, but it should be selected to be biocompatible. The probe will be discussed in more detail hereinafter with reference to Figs. 7a, 7b, 8a, and 8b.

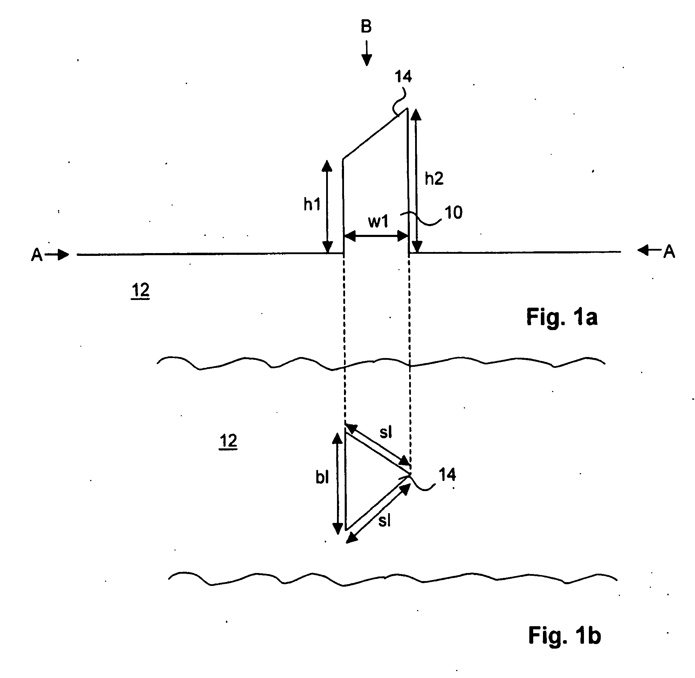

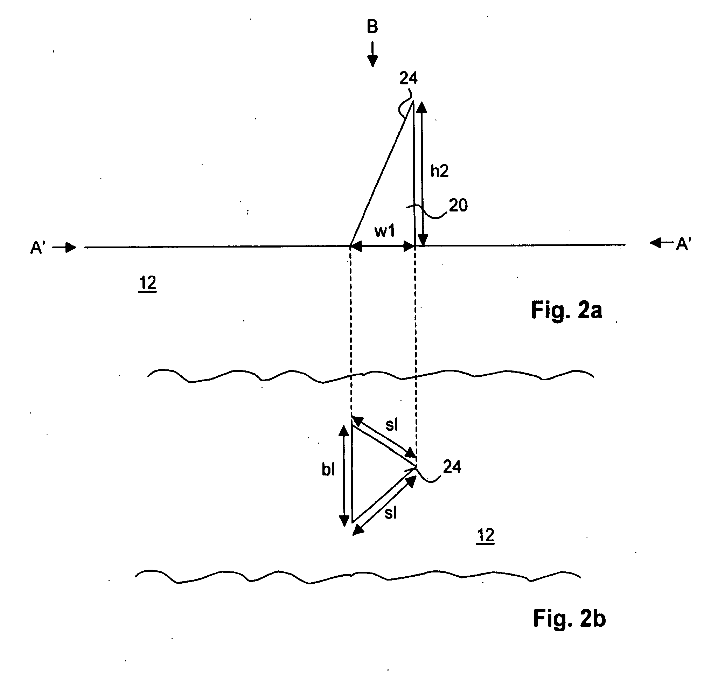

With reference now to Figs. 1a, 1b, 2a, and 2b, embodiments of microstructures or micro-needles for use in the above-mentioned electrode for the diagnosing of a diseased condition of the skin of a subject will be discussed. Only one micro-needle will be discussed in the following for the sake of simplicity, even though each electrode is provided with a plurality of such micro-needles arranged in arrays. A side view of a configuration, i.e. a cross-sectional view of a configuration along a plane perpendicular to the base substrate, and a top view of the configuration, i.e. a cross-sectional view along a plane parallel to the substrate are shown in Figs. 1a and Fig. 1b, respectively. A side view of an embodiment of the invention, i.e. a cross-sectional view of the embodiment along a plane perpendicular to the substrate, a top view of the embodiment, i.e. a cross-sectional view along a plane parallel to the substrate, are shown in Figs. 2a and Fig. 2b.

As mentioned above, each probe is provided with plurality of micro-needles, which micro-needles being integrally formed with the substrate and arranged in a laterally spaced relationship apart from each other and having a length being sufficient to penetrate the stratum corneum. Referring first to Fig. 1a, a configuration of such a micro-needle is shown along a plane perpendicular to the substrate. A micro-needle 10 is arranged on a base substrate 12, for example, a silicon wafer of SOI type. The micro-needle 10 is arranged with inclined tip portion 14 or a tip portion having an at least partially oblique shape. The tip portion 14, i.e. the oblique surface, constitutes a part of the micro-needle 10. In Fig. 1b, a top view in the direction of the arrow indicated with B along a plane substantially parallel to substrate 12 in a cross-section of the micro-needle 10 indicated by A-A in Fig. 1a, i.e. at the base of the micro-needle, is shown. As can be seen, the micro-needle 10 has a triangular cross-section in this plane. The dimensions bl and sl of the micro-needle will be substantially constant along the height h1 and will taper continuously in a direction from the surface of the base substrate 12 along the height h2-h1. It should be noted that only a part of the substrate 12 is shown in Fig. 1b. Example dimensions with reference to Figs. 1a and 1b are shown in table 1 below. With reference to Figs. 2a and 2b, an embodiment of a micro-needle for the apparatus according to the present invention is shown along a plane perpendicular to the substrate. A micro-needle 20 is arranged on a base substrate 12, for example, a silicon wafer of SOI type. The micro-needle 20 is arranged with inclined tip portion 24 or a tip portion having an oblique shape. According to this embodiment, the tip portion 24 extends to the base substrate 12. In Fig. 2b, a top view in the direction of the arrow indicated with B along a plane substantially parallel to substrate 1.2 in a cross-section of the micro-needle 20 indicated by A-A in Fig. 2a, i.e. at the base of the micro-needle, is shown. As can be seen, the micro-needle 20 has a triangular cross-section in this plane. In this case, the cross-section parallel to the substrate 12 will taper continuously in a direction from the surface of the base substrate 12. It should be noted that only a part of the substrate 12 is shown in Fig. 2b. Example dimensions with reference to Figs. 2a and 2b are also shown in table I below. For example, h1 is in the embodiment shown in Fig. 2a and Fig. 2b substantially zero.

**Table 1**

| Dimension | µm |
|---|---|
| h1 | 0 - 230 |
| h2 | 20 - 250 |
| w1 | 20 - 200 |
| b1 | 20 - 200 |
| s1 | 20 - 200 |

The fabrication of micro-needles extending from the plane of a silicon wafer is well known. See for example US 2004/0243063, "Microneedle array module and method for fabricating the same", Shuvo R., and Aaron J. F.; and US 6,334,865, "Microneedle devices and methods for manufacture and use thereof", Allen M. G., et al.

The microneedle arrays may be formed from a wafer (a substrate), where inclined surfaces are provided on the substrate by an etching process, and a mask is provided on the inclined surfaces using an appropriate patterning process (such as lithography using photo resist). The structure of the needles are then formed in a desired configuration by a second etching process. The inclined surfaces enable the manufacture of needles having an oblique end surface at the tip portion of the needle.

The wafer-like substrate may be a single crystalline silicon having surfaces exhibiting crystal directions/orientations such as <100>, <110>, etc. However, other crystalline materials that can be subjected to the same or similar processing techniques are equally well suited. In particular, a useful starting substrate is so called SOI wafers (Silicon On Insulator), since SOI wafers will provide well defined etch stop layers. Such stop layers are practical for the purpose of defining dimensions (i.e. heights, widths, depths of recesses etc.) of the structures being made, and for climinating certain unwanted side effects of etching.

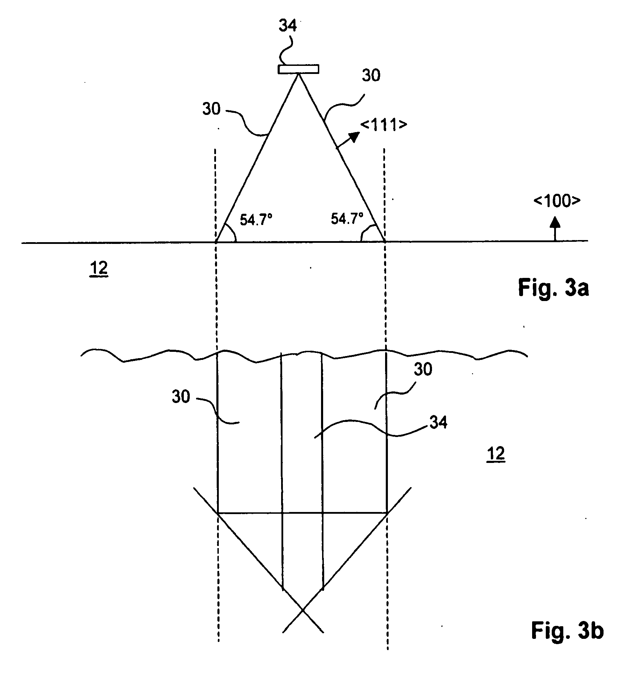

Reference is now made to Figs. 3a and 3b, Fig. 3a showing a side view of an etched wafer and Fig. 3b a top view of the same wafer. As mentioned above, an etching process to obtain the inclined surfaces 30 is first performed. Preferably, an anisotropic etching process, normally using aqueous KOH, is performed. This etching process will act on a silicon wafer 12 in which the <100> plane is horizontal, such that if a mask 34, that resists the etching medium, in the form of an elongated strip will provide a "ridge"-like structure having sloping/inclining side surfaces starting from under the mask and following the <111> plane at an angle of 54.7° down to the horizontal <100> plane. This is schematically shown in Figs. 3a and 3b. The height of the pyramidal structure will depend on the etching, for example, the etching time. If an SOI wafer with a well defined Si thickness is used, the height can be controlled very accurately, since the oxide layer will act as an etch stop.

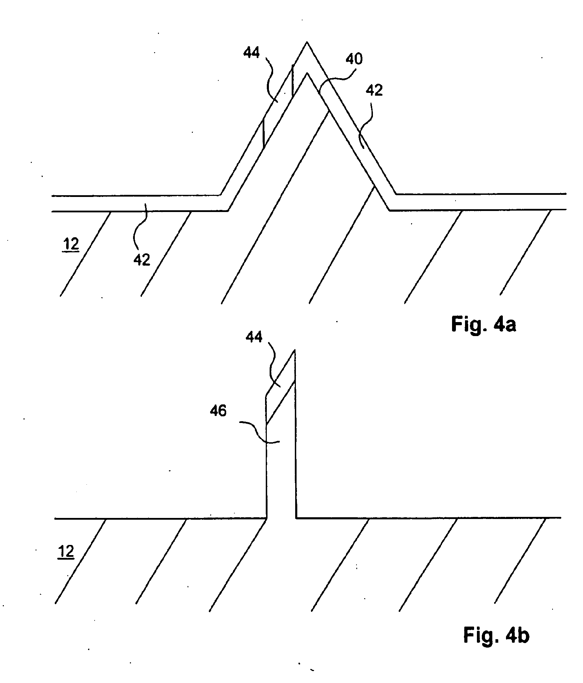

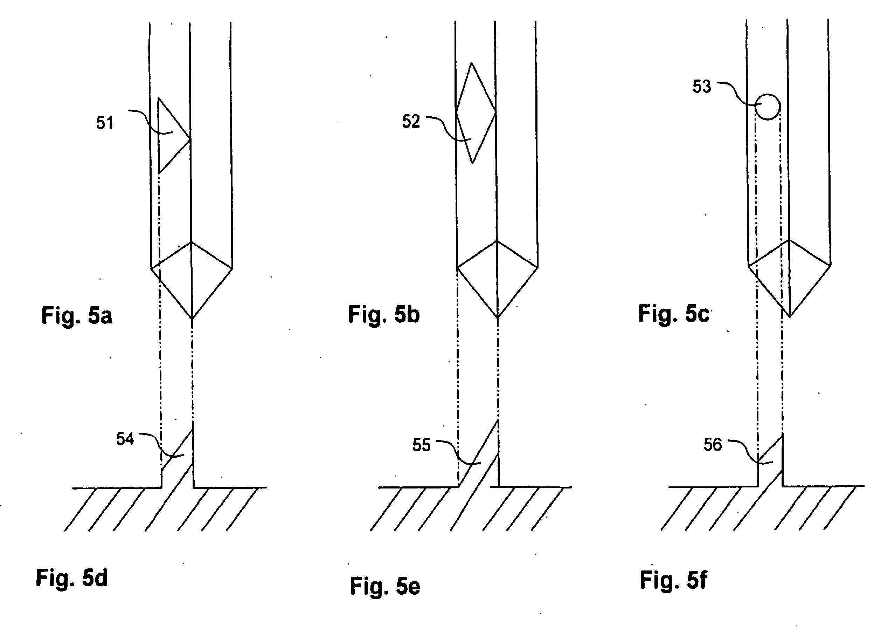

Referring now to Figs. 4a, 4b and 5a-5f, Fig 4a showing a side view of an etched wafer provided with a masking layer and Fig. 4b showing a side view of the resulting needle. Figs. 5a-5f show different mask configurations for forming needles having different structures and the resulting needle structures. As can be seen in Fig. 4a, the inclined surfaces 40 are patterned in order to define the final structures, i.e. needles having an oblique tip surface. A lithographic procedure is utilized to provide a uniformly thick masking layer 42 on an inclined surface 40. For example, a photo resist, suitably by a spraying technique in conventional manner (thus known per se), is applied such that the entire surface is covered, which is then exposed to light such that only the desired mask portions defining the structures to be made will cure 44. Thereafter, the remaining portions of resist that has not cured will be removed by dissolving/washing. The resist is shown schematically in Fig. 4a before removing the uncured portions.

An isotropic etching procedure is then performed. A preferred procedure is a Deep Reactive Ion Etching (DRIE). Thus, the result will not depend on crystal plane orientation, and, therefore, the mask will define vertical walls of the protruding structure. The opposite inclined surface, i.e. the surface that is masked to yield a protruding structure, will remain as an inclined surface, by virtue of the DRIE being isotropic. The resulting needle 46 is shown in Fig. 4b. The mask 44 has not been removed yet in Fig. 4b.

As can be seen in Figs. 5a-5f, needles having a plurality of different shapes can be formed using this method, for example, triangular, circular, square, elliptical, polygonal, etc. In Figs. 5a-5c, top views of the ridges provided with cured masks 51, 52, and 53, respectively. In Figs. 5d-5e, side views of the resulting needles are shown, where a triangular needle 54 having an oblique tip portion is shown in Fig. 5d, a rhombic needle 55 having an oblique tip portion is shown in Fig. 5e, and a circular needle having an oblique tip portion is shown in Fig. 5f.

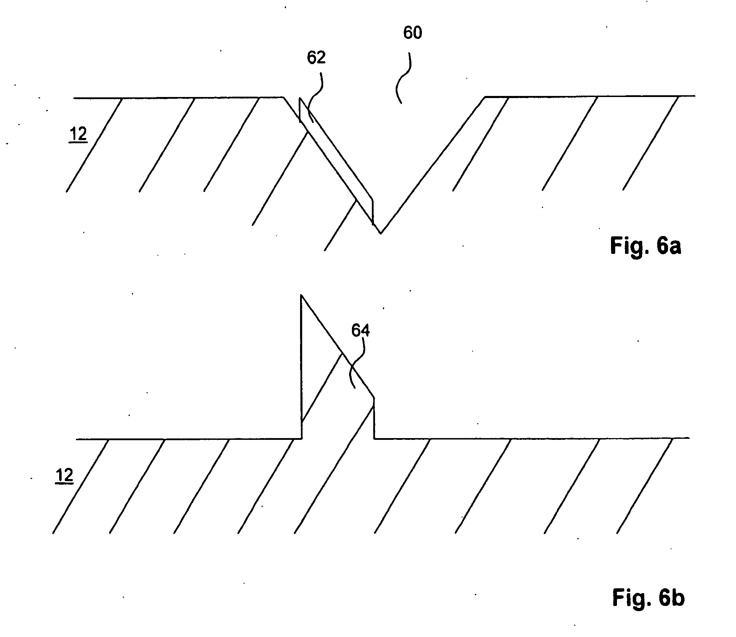

In an alternative method, the starting substrate for making protruding structures with obliquely shaped tip portions comprises V-shaped recesses 60 in the wafer instcad of the protruding "ridges" discussed above, see Figs. 6a.

These recesses are made by masking a starting wafer so as to leave elongated slits in the resist, i.e. a "negative" of the mask from the first described method. Subsequently, this masked wafer is subjected to an anisotropic etch, for example, a KOH-etch, which will act selectively on the exposed wafer material through the slits, thereby yielding a V-groove 60 wherein the inclined surfaces correspond to the <111> planes of the crystalline wafer. Similarly to the above discussed embodiment, the wafer, now comprising a plurality of V-grooves, is appropriately masked, suitably using a resist that is sprayed on for achieving a uniform layer of resist, to define the geometry of the desired structure, analogously to the first described method. In Fig. 6a the cured mask portions 62 defining the desired needle is shown. A DRIE etch follows whereby wafer material is etched away to form vertically protruding structures, e.g. needles 64.

Alternative fabrication processes employing the SOI wafer approach as discussed above is the subject matter of a copending Swedish patent application (Title: "Micro needles and applications") filed on the same day as the present application in the name of Silex AB. Also, further details of the above-described fabrication method is the subject matter of the copending Swedish patent application (Title: "Micro needles and applications") filed on the same day as the present application in the name of Silex AB. The application is published as WO 2007/07004 with SE 2005 00027 60 as priority application.

Turning now to Figs. 7a-7d, an embodiment of a probe including a removable cap provided with electrodes. The probe includes a number of electrodes, at least three, and each electrode has a spiked surface, i.e. a surface provided with an array of micro-needles in accordance with, for example, the needles discussed above with reference to Figs. 1 and 2, which permits measurements to be made at a variety of skin depths. According to the embodiment shown in Fig. 7a, the probe includes three rectangular areas or bars each bar containing an array of 57 (19 x 3) needles. However, as the skilled man realizes, the probe may include other configurations of bars or areas containing other array configurations, for example, 51 (17 x 3), 24 (2 x 12), 60 (12 x 5), or 39 (13 x 3) needles. In Figs. 7b-7d, it is shown how the removable electrode cap is attached to the probe. Thus, after the measurements have been performed on a subject, the cap comprising the electrodes can be removed and thrown away and a new cap can be attached to the probe and, accordingly, the test can be made under hygienically conditions.

In Fig. 8a the probe shown in Fig. 7a-7d is shown in cross-section and Fig. 8b is an enlarged view of the encircled portion C in Fig. 8a. The probe 80 comprises a housing 81, preferably made of a metal such as steel or another durable material, which also functions as a handle or shaft during use, for example, during measurements when the operator presses the electrode against the skin of a subject. A removable cap 82 can be releasable attached to an end of the probe 80, which is shown in Figs. 7b-7d. Preferable, the cap 82 is made of a material such as plastic, which enables a user of the probe to thread the cap 82 on the tip portion of the housing 81. The removable cap 82 is provided with electrodes 83a-83c arranged on a base substrate 86. In this example, three electrodes 83a-83c each comprising an array of 57 (19 x 3) micro-needles is arranged on the cap 82. The cap 82 is disposable for hygienically reasons. Furthermore, as can be seen in Fig. 8b, the cap 82 comprises connection means 84 including terminals, in this case six terminals, adapted to make contact with corresponding connection means 85 including terminals arranged in the housing 81, in this case six terminals, of the probe 80 when the cap 82 is fully attached to the probe 80. Thereby, the electrodes 83a-83c of the cap 82 can be supplied with electrical current.

In Fig. 8a and 8b, the electrodes 83a- 83c are shaped as rectangular bars but, as the skilled man in the art realizes, other topological shapes compatible with the essential features are also conceivable. For example, the electrodes may be designed as C-shaped or concentric. Additional electrodes carrying guard, signal ground, driven guard, etc. may also be arranged. Cabling and shielding may be arranged in accordance with established engineering practice in order to minimize electromagnetic interference. Moreover, the design may also be adapted to conform to local safety regulations.

As discussed above, the micro-needles are laterally spaced apart from each other and having a length being sufficient to penetrate the stratum corneum. The probe is adapted to be placed against a skin surface of the subject such that the micro-needles penetrate the stratum corneum. Preferably, the skin surface sites are soaked prior to each impedance measurement using, for example, a 0.9 % saline solution. The sites can be soaked, for example, for 60 seconds prior to the measurement.

The apparatus according to the present invention is adapted to pass an electrical current through the electrodes to obtain a spectrum/range of values of skin impedance and to use reference data to determine whether the obtained impedance value indicates the diseased condition. The electrical current has a frequency between about 10 Hz and about 10 MHz.

The apparatus without the micro-needled probe is known as the SciBase II depth selective spectrometer, may be obtained from SciBase AB of Stockholm, Sweden. The pin assignment for the probe connected may be as follows:
1. <STAR> button.
2. sense (first electrode illustrated in Fig. 8(b) indicated by reference numeral 83a; use coaxial (conventional probe) screen 3).
3. gnd (for sense).
4. near exciter ( second (middle) electrode illustrated in Fig. 8(b) indicated by reference numeral 83c; use coaxial (conventional probe) screen 5).
5. gnd (for near injection).
6. gnd.
7. far exciter (third (right-most) electrode illustrated in Fig. 8(b) indicated by reference numeral 83b; use coaxial (conventional probe) screen 8).
8. gnd (for far injection).
9. chassis.
10. reserved.
11. reserved.
12. gnd.
13. gnd.
14. charger.

Preferably, the impedance was measured using the SciBase II depth selective spectrometer at 35 logarithmically distributed frequencies from 1 kHz to 2,5 MHz at five depth settings. In this embodiment, ten frequencies per decade are used.

The signal is voltage driven signal and has a signal strength preferably within a range uf 0 - 50 mV rms, i.e. below the resting potential of a living cell of a human being, which is approximately 70 mV.

Each micro-needle may be at least 20, or at least 30, or at least 40, or at least 50, or at least 60, or at least 70, or at least 80, or at least 90 µm in length. Additionally, each micro-needle may be up to 250, or up to 240, or up to 230, or up to 220, or up to 210, or up to 200, or up to 190, or up to 180, or up to 170, or up to 160, or up to 150, or up to 140, or up to 130, or up to 120, or up to 110, or up to 100 µm in length.

According to an alternative configuration, each micro-needle has a thickness of about 30 micrometer.

According to the invention, the probe comprises three electrodes, wherein a first electrode and a second electrode being laterally spaced apart a first distance from each other and the first and a third electrode being laterally spaced apart a second distance from each other. This is illustrated in Fig. 8b, where the sense electrode 83a is spaced apart from the near exciter electrode 83c and the far exciter electrode 83b, respectively. The first distance and the second distance are different from each other. The first distance is between about 0.1 mm and about 40 mm and the second distance is between about 1 mm and about 50 mm. The apparatus is adapted to gradually or stepwise change a proportion of a potential between the first and the second electrode and the first and the third electrode to obtain first and second values of skin impedance. Specifically, the first distance is between about 0.1 mm and about 40 mm, or between about 0.1 mm and about 30 mm, or between about 0.1 mm and about 25 mm, or between about 0.1 mm and about 20 mm, or between about 0.1 mm and about 15 mm, or between about 0.2 mm and about 10 mm, or between about 0.2 mm and about 5 mm, or between about 0.2 mm and about 3 mm, or between about 0.2 mm and about 2 mm, or between about 0.2 mm and about 1.5 mm, or between about 0.2 mm and about I mm, or between about 0.2 mm and about 0.5 mm. In addition, the second distance is between about I mm and about 50 mm, or between about 1 mm and about 40 mm, or between about 1 mm and about 30 mm, or between about I mm and about 15 mm, or between about I mm and about 10 mm, or between about 1 mm and about 9 mm, or between about 1 mm and about 8 mm, or between about I mm and about 7 mm, or between about 2 mm and about 8 mm, or between about 3 nun and about 7 mm, or between about 4 mm and about 7 mm, or between about 4 mm and about 6 mm, or about 5 mm.

Furthermore, each electrode may comprise at least two micro-needles, or at least three micro-needles, or at least four micro-needles, or at least five micro-needles, or at least six micro-needles, or at least seven micro-needles, or at least eight micro-needles, or at least nine micro-needles, or at least ten micro-needles, or at least twelve micro-needles, or at least fifteen micro-needles, or at least eighteen micro-needles, or at least twenty micro-needles, or at least twenty-five micro-needles, or at least thirty micro-needles, or at least thirty-five micro-needles, or at least fifty micro-needles. As mentioned above, the probe illustrated in Fig. 7 and 8 includes three rectangular areas or bars, each bar containing an array of 57 (19 x 3) micro-needles, but, as the skilled man realizes, a number of other array configurations, for example, arrays of 39 (13 x 3), 45 (15 x 3), 24 (12 x 2), 48 (4 x 12), 65 (13 x 5), or 51 (17 x 3) micro-needles are also conceivable. According to alternatives, the micro-needles are arranged in a non-linear fashion. Also electrodes having only one row of micro-needles are conceivable.

Impedance measurements using the probe according to the present invention of a subject suffering from basal cell carcinoma or malignant melanoma: at a first site of normal (unaffected skin); and at a second site of diseased skin, may be performed in accordance with the approach described in Emtestam I. Nicander I, Stenström M, Ollmar S. "Electrical impedance of nodular basal cell carcinoma: a pilot study", Dermatology 1998; 197: 313-316, and Kapoor S. "Bioelectric impedance techniques for clinical detection of skin cancer using simple electrical impedance indices", Skin Res Technol 2003; 9: 257-261, and Beetner DG, Kapoor S, Manjunath S, Zhou X, Stoecker WV "Differentation among basal cell carcinoma, benign lesions, and normal skin using electric impedance", IEEE Trans Biomed End 2003; 50: 1020-1025. However, the measurements described in these references were obtained using a conventional probe and an early version of the impedance spectrometer.

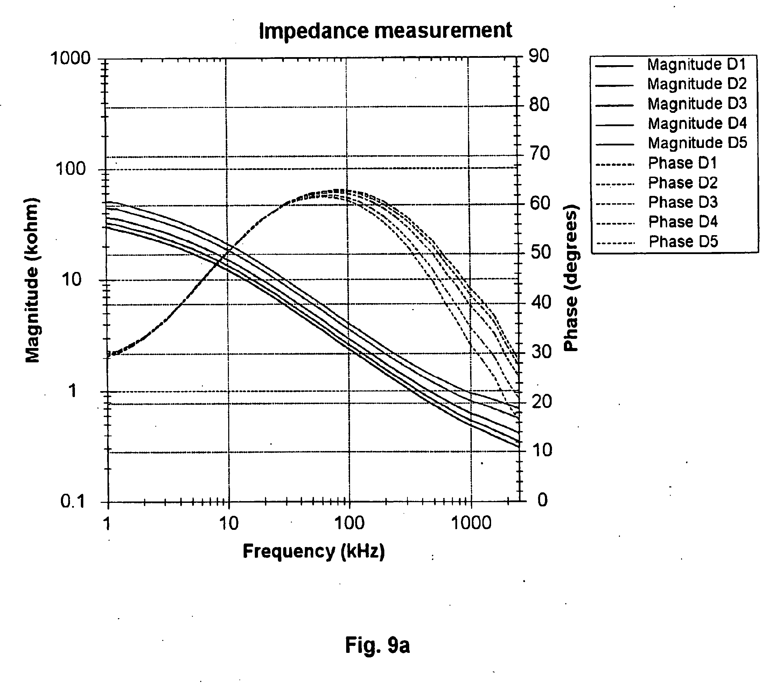

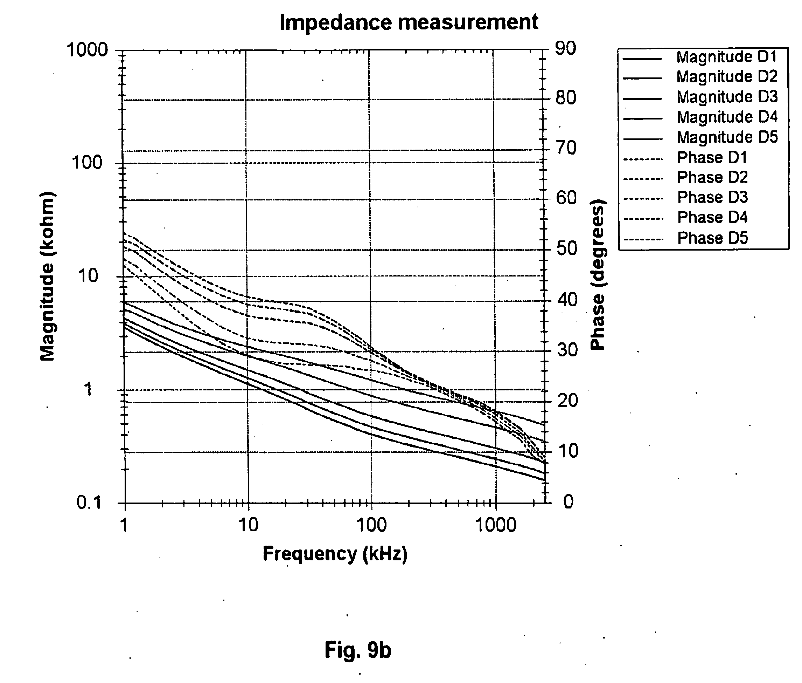

In Figs. 9a and 9b results from measurements performed using a probe with micro-needles designed in accordance with the configuration described with reference to Fig. 1a and 1b, i.e. with a triangular.cross-section, of a subject suffering from basal cell carcinoma are shown. In fig. 9a measurements at a first site of normal (unaffected skin), i.e. a reference site, and in Fig. 9b measurements at a second site of diseased skin, are shown, respectively. As can be seen, there is a significant deviation of the impedance, both with respect to the phase and the magnitude, between measurements performed on a site of unaffected skin and measurements performed on a site of diseased skin.

It is desirable to detect and remove skin cancers as early as possible. As such, precursors of skin cancer, such as, for example, actinic keratose (a precursor of squamous cell carcinoma) and dysplastic nevi (a precursor of malignant melanoma), as well as other lesions that may be mixed up with various cancers unless surgery and histological evaluation of the catch is made, can be detected using impedance measurements of the present invention in the manner described herein.

In addition, the present invention may be used for measuring and/or monitoring and/or detecting biological conditions, for example, changes of skin properties of a subject, or changes of tissue properties of a subject. Moreover, the present invention may also be used for measuring and/or monitoring and/or detecting biological conditions of tissue of an organ, such as a kidney.

Although exemplary embodiments of the present invention has been shown and described, it will be apparent to those having ordinary skill in the art that a number of changes, modifications, or alterations to the inventions as described herein may be made. Thus, it is to be understood that the above description of the invention and the accompanying drawings is to be regarded as a non-limiting example thereof and that the scope of protection is defined by the appended patent claims.

## Claims

1. A medical apparatus for the diagnosing of a diseased condition of the skin of a subject, comprising an electrically conducting probe (80) including a plurality of electrodes (83a - 83c), each electrode (83a - 83c) comprising a plurality of micro-needles (20), wherein said probe (80) is adapted to be placed against a surface of the subject such that said micro-needles (20) penetrate the stratum corneum, wherein said medical apparatus is adapted to initiate an impedance measurement session including passing an electrical current through the electrodes (83a - 83c) to obtain values of skin impedance, and use reference data to determine whether the obtained impedance values indicate the diseased condition, wherein
the micro-needles (20) of a first electrode (83a) and a second electrode (83b) being laterally spaced apart a first distance from each other and the micro-needles (20) of the first and a third electrode (83a, 83c) being laterally spaced apart a second distance from each other, said apparatus being adapted to gradually change a proportion of a potential between the first and the second electrode (83a, 83b) and the first and the third electrode (83a, 83c) to obtain first and second values of skin impedance or a mixture of values of skin impedance between values obtained using the first and second electrode and values obtained using the first and third electrode; and wherein
each electrode comprises (83a - 83c) a base substrate (12; 86), said micro-needles (20) being integrally formed with said base substrate (12; 86) and arranged in a laterally spaced relationship apart from each other and having a length being sufficient to penetrate the stratum corneum, said microneedles (20) having a substantially triangular cross-section parallel to the base substrate (12; 86), and said micro-needles (20) being arranged with an oblique surface (24) such that the cross-section parallel to the base substrate (12; 86) tapers continously from the base substrate (12; 86).

2. The medical apparatus according to claim 1, wherein each of said micro-needles (20) has a length being sufficient to penetrate below the skin surface to the Stratum Germinativum or through the Stratum Corneum into the living Epidermis but not into the Dermis.

3. The medical apparatus according to claim I, wherein said medical apparatus is adapted to, at placing against a surface of a subject, provide an indication that said micro-needles have penetrated below the skin surface to the Stratum Germinativum or through the Stratum Corneum into the living Epidermis but not into the Dermis.

4. The medical apparatus according to claim 3, wherein said medical apparatus is adapted to, at placing against a surface of a subject, pass an electrical current having a frequency at a lower end of a predetermined frequency spectrum through the electrodes (83a - 83c) in order to obtain values of skin impedance, compare said obtained impedance values with a predetermined reference impedance value for said frequency and determine that said micro-needles (20) have penetrated the stratum corneum if said obtained impedance value is below said reference value.

5. The medical apparatus according to any one of preceding claims, wherein the diseased condition is cancer, preferably skin cancer.

6. The medical apparatus according to claim 5, wherein skin cancer is a basal cell carcinoma, a malignant melanoma, a squamous cell carcinoma, or precursors of such lesions.

7. The medical apparatus according to any one of preceding claims, wherein said electrical current has a frequency in a predetermined frequency spectrum between about 10 Hz and about 10 MHz.

8. The medical apparatus according to claim 7, wherein said apparatus is adapted to pass said electrical current through the electrodes at a plurality of logarithmically distributed frequencies, said frequencies having a range from 1 kHz to 2.5 MHz.

## Patentansprüche

1. Medizinisches Gerät zur Diagnose eines Krankheitszustandes der Haut einer Person, das eine elektrisch leitende Sonde (80) umfasst, die mehrere Elektroden (83a - 83c) enthält, jede Elektrode (83a - 83c) mehrere Mikronadeln (20) aufweist, wobei die Sonde (80) dafür eingerichtet ist, gegen eine Fläche von der Person derart gepresst zu werden, dass die Mikronadeln (20) das Stratum corneum durchdringen, wobei das medizinische Gerät derart eingerichtet ist, eine Impedanz-Messungssitzung zu beginnen, welche das Durchleiten eines elektrischen Stroms durch die Elektroden (83a - 83c) umfasst, um Werte der Hautimpedanz zu erhalten, und Referenzdaten zu verwenden, um zu bestimmen, ob die erhaltenen Impedanzwerte auf den Krankheitszustand hinweisen, wobei die Mikronadeln (20) einer ersten Elektrode (83a) und einer zweiten Elektrode (83b) zur Seite hin mit einem ersten Abstand voneinander beabstandet sind und die Mikronadeln (20) der ersten und einer dritten Elektrode (83a, 83c) zur Seite hin mit einem zweiten Abstand voneinander beabstandet sind, wobei das Gerät dafür eingerichtet ist, schrittweise einen Anteil einer Spannung zwischen der ersten und der zweiten Elektrode (83a, 83b) und der ersten und der dritten Elektrode (83a, 83c) zu ändern, um erste und zweite Werte der Hautimpedanz zu erhalten oder eine Mischung aus Werten der Hautimpedanz zwischen Werten zu erhalten, die unter Verwendung der ersten und zweiten Elektrode erhalten wurden und Werten, die unter Verwendung der ersten und dritten Elektrode erhalten wurden; und wobei
jede Elektrode (83a - 83c) ein Basissubstrat (12, 86) aufweist, die Mikronadeln (20) integral mit dem Basissubstrat (12, 86) ausgeformt und in einem zur Seite hin beabstandeten Verhältnis zueinander angeordnet sind und eine Länge aufweisen, die ausreichend ist, um das Stratum corneum zu durchdringen, die Mikronadeln (20) einen im Wesentlichen dreieckigen Querschnitt parallel zu dem Basissubstrat (12, 86) aufweisen und die Mikronadeln (20) mit einer schrägen Fläche (24) derart angeordnet sind, dass der Querschnitt parallel zum Basissubstrat (12, 86) sich kontinuierlich von dem Basissubstrat (12, 86) her verjüngt.

2. Medizinisches Gerät gemäß Anspruch 1, wobei jede der Mikronadeln (20) eine Länge aufweist, die ausreichend ist, unter die Hautoberfläche bis zum Stratum germinativum oder durch das Stratum corneum bis in die lebende Epidermis, aber nicht bis in die Dermis einzudringen.

3. Medizinisches Gerät gemäß Anspruch 1, wobei das medizinische Gerät dafür eingerichtet ist, bei Platzieren gegen eine Fläche von einem Person, eine Anzeige bereitzustellen, dass die Mikronadeln die Hautoberfläche bis zum Stratum germinativum oder durch das Stratum corneum bis in die lebende Epidermis, aber nicht bis in die Dermis durchdrungen haben.

4. Medizinisches Gerät gemäß Anspruch 3, wobei das medizinische Gerät dafür eingerichtet ist, bei Platzieren gegen eine Fläche von einem Person, einen elektrischen Strom mit einer Frequenz an einem unteren Ende eines vorher festgelegten Frequenzspektrums durch die Elektroden (83a - 83c) zu leiten, um Werte für die Hautimpedanz zu erhalten, die erhaltenen Impedanzwerte mit einem vorgegebenen Referenzimpedanzwert für diese Frequenz und feststellen, dass die Mikronadeln (20) das Stratum corneum durchdrungen haben, wenn der erhaltene Impedanzwert unterhalb des Referenzwertes ist.

5. Medizinisches Gerät gemäß einem der vorhergehenden Ansprüche, wobei der Krankheitszustand Krebs, vorzugsweise Hautkrebs ist.

6. Medizinisches Gerät gemäß Anspruch 5, wobei der Hautkrebs ein Basalzellkarzinom, ein malignes Melanom, ein squamöses Zellkarzinom ist oder Vorstufen solcher Läsionen sind.

7. Medizinisches Gerät gemäß einem der vorhergehenden Ansprüche, wobei der elektrische Strom eine Frequenz in einem vorgegebenen Frequenzspektrum zwischen ungefähr 10 Hz und ungefähr 10 MHz aufweist.

8. Medizinisches Gerät gemäß Anspruch 7, wobei das Gerät dafür eingerichtet ist, den elektrischen Strom durch die Elektroden mit mehreren logarithmisch verteilten Frequenzen durchzuleiten, wobei die Frequenzen einen Bereich von 1 kHz bis 2,5 MHz aufweisen.

## Revendications

1. Appareil médical destiné au diagnostic d'un état pathologique de la peau d'un sujet, ledit appareil comprenant une sonde électro-conductrice (80) comprenant une pluralité d'électrodes (83a - 83c), chaque électrode (83a - 83c) comprenant une pluralité de micro-aiguilles (20), dans lequel ladite sonde (80) est apte à être placée contre une surface du sujet de telle sorte que lesdites micro-aiguilles (20) pénètrent la couche cornée, dans lequel ledit appareil médical est apte à initier une session de mesure d'impédance comprenant le passage d'un courant électrique dans les électrodes (83a - 83c) pour obtenir des valeurs d'impédance de la peau, et à utiliser des données de référence pour déterminer si les valeurs d'impédance obtenues indiquent ou non l'état pathologique, dans lequel
les micro-aiguilles (20) d'une première électrode (83a) et d'une deuxième électrode (83b) sont latéralement espacées d'une première distance les unes des autres et les micro-aiguilles (20) de la première électrode et d'une troisième électrode (83a, 83c) sont latéralement espacées d'une seconde distance les unes des autres, ledit appareil étant apte à graduellement modifier une proportion d'un potentiel entre les première et deuxième électrodes (83a, 83b) et les première et troisième électrodes (83a, 83c) pour obtenir des première et seconde valeurs d'impédance de la peau ou un mélange de valeurs d'impédance de la peau entre les valeurs obtenues en utilisant les première et deuxième électrodes et les valeurs obtenues en utilisant les première et troisième électrodes ; et dans lequel
chaque électrode (83a - 83c) comprend un substrat de base (12 ; 86), lesdites micro-aiguilles (20) formant partie intégrante dudit substrat de base (12 ; 86) et étant configurées dans une relation d'espacement latéral les unes par rapport aux autres et ayant une longueur suffisante pour pénétrer la couche cornée, lesdites micro-aiguilles (20) ayant une section transversale sensiblement triangulaire parallèle au substrat de base (12 ; 86), et lesdites micro-aiguilles (20) étant configurées avec une surface oblique (24) de manière à ce que la section transversale parallèle au substrat de base (12 ; 86) rétrécisse continuellement à partir du substrat de base (12 ; 86).

2. Appareil médical selon la revendication 1, dans lequel chacune desdites micro-aiguilles (20) a une longueur suffisante pour pénétrer sous la surface de la peau jusqu'à la couche germinative ou traverser la couche cornée jusqu'à l'épiderme vivant mais pas jusqu'au derme.

3. Appareil médical selon la revendication 1, dans lequel ledit appareil médical est apte, lorsqu'il est placé contre une surface d'un sujet, à indiquer que lesdites micro-aiguilles ont pénétré sous la surface de la peau jusqu'à la couche germinative ou ont traversé la couche cornée jusqu'à l'épiderme vivant mais pas jusqu'au derme.

4. Appareil médical selon la revendication 3, dans lequel ledit appareil médical est apte, lorsqu'il est placé contre une surface d'un sujet, à faire passer dans les électrodes (83a -83c) un courant électrique ayant une fréquence au niveau d'une extrémité inférieure d'un spectre de fréquences prédéterminé de manière à obtenir des valeurs d'impédance de la peau, à comparer lesdites valeurs d'impédance obtenues à une valeur d'impédance de référence prédéterminée pour ladite fréquence et à déterminer si lesdites micro-aiguilles (20) ont ou non pénétré la couche cornée si ladite valeur d'impédance obtenue est inférieure à ladite valeur de référence.

5. Appareil médical selon l'une quelconque des revendications précédentes, dans lequel l'état pathologique est un cancer, de préférence le cancer de la peau.

6. Appareil médical selon la revendication 5, dans lequel le cancer de la peau est un carcinome basocellulaire, un mélanome malin, un carcinome à cellules squameuses, ou des précurseurs de ces lésions.

7. Appareil médical selon l'une quelconque des revendications précédentes, dans lequel ledit courant électrique a une fréquence dans un spectre de fréquences prédéterminé compris entre environ 10 Hz et 10 MHz.

8. Appareil médical selon la revendication 7, dans lequel ledit appareil est apte à faire passer ledit courant électrique dans les électrodes à une pluralité de fréquences réparties de manière logarithmique, lesdites fréquences se trouvant dans une plage de 1 kHz à 2,5 MHz.
